# EUROPEAN PATENT SPECIFICATION

(11) **EP 4 480 422 B1**
(45) Date of publication and mention of the grant of the patent: **14.05.2025**
(21) Application number: 23181145.6
(22) Date of filing: 23.06.2023
(51) Int. Cl.: A61B 7/00, A61B 7/02, A61B 7/04

(54) **MULTI-SENSOR AUSCULTATION DEVICE AND ANALYSIS METHOD THEREFOR**
AUSKULTATIONSVORRICHTUNG MIT MEHREREN SENSOREN UND ANALYSEVERFAHREN DAFÜR
DISPOSITIF D'AUSCULTATION À CAPTEURS MULTIPLES ET PROCÉDÉ D'ANALYSE ASSOCIÉ

(43) Date of publication of application: 25.12.2024
(73) Proprietor: Lynx Health Science GmbH, 57439 Attendorn (DE)
(72) Inventor: Klum, Michael, 13581 Berlin (DE)
(74) Representative: Gulde & Partner

(56) References cited:
- US-A1- 2022 142 600
- US-B1- 9 924 921

## Description

### Field of the Invention

The invention relates to the field of monitoring the activity of a body part of a subject.

### Technological Background

Monitoring the activity of a body part is essential for assessing the well-being or the fitness of a subject, such as a human being, and for determining a possible medical procedure to be administered thereto. Such procedures could be beneficial and prescribed to address or assess a specific concern, during a fitness/well-being assessment, or following a medical procedure for assessing the results of that procedure or the recovery process therefrom.

When assessing a body part of a person by auscultation of that body part, the measurements at different auscultation areas are usually performed sequentially, since the person assessing that body part cannot analyze multiple signals simultaneously. With the introduction of automated devices for body part assessment, the manner of human assessment has always been mimicked, so that sequential or at least independent measurement and assessment of auscultation signals has been maintained.

Advanced assessment devices use a combination of auscultation and other biomedical measurement methods together, but either auscultation measurements at singular auscultation sites are taken or, to anticipate repositioning, auscultation measurements at multiple auscultation sites are taken simultaneously but assessed independently. However, repositioning of the sensors is burdensome for the users and an individual assessment of several sensors does not take advantage of combined information that may be derivable.

Thus, there is a need in the prior art for measuring devices that can simply and less burdensome be used by users and that allows to simultaneously measure and assess a plurality of auscultatory and other biomedical signals and combine all the information in a beneficial manner.

US 9 924 921 B1 discloses a method for monitoring joint performance using a plurality of auscultation sensors and their respective location, but fails to evaluate the acquired auscultation data with other biomedical signals. US 2022/142600 A1 relates to four-dimensional sound maps from heart auscultation, whereby the three-dimensional spatial distribution and the one-dimensional temporal variation of the acoustic sources are displayed, but no other biomedical signals are used for conjoint evaluation.

### General Description of the Invention

The present invention discloses a multi-sensor auscultation device for measuring and/or monitoring a body part of a subject, comprising a plurality of surface vibration sensitive phono-gram sensors, PG sensors, each configured to measure an acoustic activity of the body part and each to provide a PG signal indicative of the acoustic activity of the body part, wherein the PG sensors are adapted to be arranged on the body part to cover different distinct auscultation areas of said body part, further at least one other biomedical sensor, BIO sensor, configured to measure in dependence of the sensor type a corresponding activity of the body part and each providing at least one signal indicative of the corresponding activity of the body part and further a signal processing unit, configured to obtain the signals from the sensors and to conjointly perform signal source specific analysis by combining the information of the PG signals with a respective placement information where the PG sensors are arranged on the body part and the information of the BIO signal.

As used herein, the term "subject" refers to a body on which the measurements are applied, such as a human being or an animal, such as a mammal, reptile, or bird. The terms "human", "subject", "mammal", "body", "animal", "reptiles", and "birds" are interchangeably used.

As used herein, a body part refers to a tissue, a combination of tissues, an implanted tissue, an implanted device, an implanted artificial organ, or a combination thereof, in or on the body of said subject. Commonly, the body part reflects certain physiological functions such as cardiac functions, respiratory functions, neurological functions, digestive functions, vascular functions, structural functions, or the like. Examples for body parts comprise the heart, lung, joints such as the hip, knee, shoulder, finger joints, or the like, the gastrointestinal tract, larynx, esophagus, spine, or certain muscles or muscle groups, or the like, or certain parts thereof. In particular, a multi-sensor auscultation device according the invention may be configured to measure an activity of a specific body part directly or in a region related to said specific body part, i.e. in a respective body part region comprising different distinct auscultation areas of said body part.

As used herein, the term "signal" refers to a discretely sampled time signal, preferably stored digitally. According to some embodiment of the present disclosure this signal is an acoustic signal or a biomedical signal, such as an electric signal like an electrocardiography (ECG), electromyography (EMG), electroencephalography (EEG) or electrooculography (EOG) signal, a photoplethysmography (PPG) signal, an impedance signal like an impedance plethysmography (IPG), bioimpedance spectroscopy (BIS), impedance cardiography (ICG), impedance pneumography (IP) or electrical impedance tomography (EIT) signal, an oxygen saturation (SpO2) signal, a blood pressure signal, a glucose signal, a temperature signal, a respiratory signal, a blood flow signal, an accelerometer signal, or the like.

As used herein, the term "sensor" refers to a module configured to sense or measure a designated property, such as surface vibrations, surface electrical potential differentials, impedance, or the like, wherein the sensing activity is performed directly by the sensor, for example when the sensor includes an interface with the body of the subject, or indirectly, for example when the sensor is configured to be connected to an interface medium, such as an electrode, a membrane or a part of the device case, which is placed on the body of the subject.

As used herein, the term "PG sensor" refers to a sensor configured to measure vibrations, such as acoustic and/or mechanical vibrations caused by or resulting from the activity of one or more body parts of the subject or by other factors, such as bodily fluids including blood, urine, stomach acid, salvia, or the like, non-bodily fluids including swallowed water or the like, non-bodily solids including swallowed food or the like, bodily gases including digestive byproducts or the like, non-bodily gases including air in the lungs or the like, artificial components such as implanted components, including prosthetic heart valves, joints, catheters, or the like.

According to some embodiments, each of the PG sensors includes, or is connected to, an interface configured to obtain, and optionally amplify, mechanical/acoustic vibrations/auscultation, such as a membrane, and to transform the obtained vibrations/auscultation to an electrical PG signal, wherein the multiple sensors might be implemented differently.

According to some embodiments, the PG sensor is configured to transform the obtained vibrations to an electrical PG signal using an accelerometer, a gyrometer, a magnetometer, a pressure-resistive sensor, a condenser sensor using a pressure-capacitor, such as a parallel-plate capacitor configured to convert acoustic pressure into displacement and to act as a moving plate of the capacitor, a piezoelectric sensor, a solid-state acoustic detector, microelectromechanical systems (MEMS) or the like, wherein the multiple sensors might be implemented differently.

As used herein, the wording "an acoustic activity of the body part" refers to a mechanical activity, vibrational activity, gas flow activity, fluid flow activity, or the like or any combination thereof.

As used herein, the term "indicative of" refers to the dependence of the information obtained from the signal on the activity, condition or state of the body part being monitored. Particularly, a signal can be indicative of activities of multiple body parts since the monitored/target body part resides in the vicinity of other body parts and could be affected by the activities thereof. Additionally, even when the activity of the measured body part is not directly affected by the activities of other body parts, the activities of other body parts can affect the measured signal.

As used herein, the term "BIO sensor" refers to a sensor configured to measure a biomedical activity of the body part according to the type of the BIO sensor, which is different from the PG sensor. In one embodiment, the BIO sensor is referring to a surface electrical potential differentials sensor, configured to measure an electrical activity of the body part and/or an impedance sensor, configured to measure an electrical impedance of the body part. According to some embodiments, the BIO sensor could comprise photoplethysmography (PPG) sensors, electromyography (EMG) sensors, electrocardiogram (ECG) sensors, electroencephalogram (EEG) sensors, electrooculography (EOG sensors), or any other electric potential sensor, impedance plethysmography (IPG) sensors, bioimpedance spectroscopy (BIS) sensors, impedance cardiography (ICG) sensors, impedance pneumography (IP) sensors, electrical impedance tomography (EIT) sensors, or any other impedance sensor, oxygen saturation (SpO2) sensors, blood pressure sensors, glucose sensors, temperature sensors, respiratory sensors, blood flow sensors, accelerometers, or the like. For different sensor types, different requirements will be necessary such as different amounts of sensors, different placements of the sensors, and/or complementary aids, such as electrical components or interfaces.

According to some embodiments, one or more of the sensors in the device includes an interface medium configured to be placed on the body of the subject, such as on a certain portion of the skin of the subject. According to some embodiments, one or more of the sensors includes a connector configured to achieve electrical, capacitive coupling, magnetic, and/or mechanical connection with an interface medium. Advantageously, such a configuration facilitates the use of replaceable interface media, such as replaceable electrodes for improving hygiene and/or measurement quality.

As used herein the term "auscultation area" refers to a specific region in or on the subject's body from which internal sounds of the body are easily perceived. These specific areas are identified based on the location of underlying organs, structures, or tissues. For example, the common auscultation areas of the heart are located on the chest and correspond to the four valves of the heart, while similarly auscultation areas of the lungs are located on the chest and back and correspond to the lobes of the lungs. At an auscultation area of a body part, a clear and distinct acoustic signal of an auscultatory source must be perceptible during auscultation. In some cases, the auscultation areas of a body part are known, in some cases they are not, and in other cases there are additional previously unknown auscultation areas. In some cases, the sound of one auscultation area is indistinguishable from that of another auscultation area if the corresponding auscultatory source is not present or does not produce a sound, e.g., at times a joint may produce a sound that can be easily auscultated, but in another case the anatomical structure is such that no sound is produced. A classic array of PG sensors in a regular or random pattern will in most cases not cover different distinct auscultation areas as required in the invention, as this usually requires careful and considered placement of PG sensors or a specific selection of channels of a PG sensor array.

As used herein, the terms "conjoint" or "conjointly" in relation to the analysis of the PG signals, and the BIO signal refer to performing analysis, such that the results of the analysis are based on all of the PG signal, and the BIO signal inclusively and the exclusion of one of the PG signals and the BIO signal would not result in the same outcome of the analysis. For example, a conjoint analysis of the PG signal, and the BIO signal has an outcome that is different from an analysis of any one or combination of a subgroup of the PG signals and the BIO signal. The difference in the outcome of a conjoint analysis of all of the PG signals and the BIO signal can be a difference in a confidence value, such as statistical significance, noise levels, or the like, and/or can be a difference in an actual value of the outcome of the analysis.

According to some embodiments, conjoint analysis can be achieved by inputting all of the PG signals and the BIO signal to a computational model for generating an outcome based on all of the PG signals and the BIO signal. Alternatively, according to some embodiments, one or more of the PG signals and the BIO signal are inputted to a first computational model, and the outcome thereof is inserted into at least one other computational model that receives as input at least some of the remaining signals. According to some embodiments, the at least one other computational model receives as an input the outputs from earlier-stage computational models. According to some embodiments, for the conjoint analysis of signals, it is necessary to have synchronously measured signals or at least signals that have been synchronized through various methods such as timestamps, fiducial point alignment, correlation alignment, feature alignment, or the like. The synchronous nature of the signals ensures that the signals under analysis are aligned in time and can be properly compared and analyzed conjointly and is therefore required in most cases.

As used herein, the term "signal source specific analysis" refers to a method for analyzing signals originating from different sensors, taking into account the distinct position of these sensors, and thus the possible source of the respective signals. In the context of the present invention, the signal source specific analysis comprises the conjoint analysis of PG signals detected by sensors placed at specific auscultatory areas combined with a respective placement information of these specific auscultatory areas and at least one other BIO signal. According to some embodiments, the PG sensors for the PG signals are positioned to measure auscultatory signals comprising different auscultatory signal components originating from the respective auscultatory source whose activity is best measurable from the respective PG sensor location. Each PG signal may be designed to have the strongest possible signal component for one certain distinct signal source, even though in some cases this signal component might be smaller than the superimposed sounds of other sources or even undistinguishable from other sounds. Hence, the signal source specific analysis may take into account this specific design benefit and may be aimed to obtain more information from these PG signals, the respective placement information of the PG sensors and the BIO signal than by analyzing the individual signals or without knowledge of the respective placement information.

According to some embodiment, the signal source specific analysis comprises an analysis of the signals with respect to the placement information, wherein the signal is raw or filtered and is analyzed completely or only in parts. According to some embodiment, the signal source specific analysis comprises an analysis of certain features with respect to the placement information, wherein the features are derived from the signals. According to some embodiment, the signal source specific analysis generates a signal, value, index, segmentation, detection, timing, percentage, or the like, or any combination and multiplication thereof.

According to some embodiment, the signal source specific analysis comprises a filtering method of the signals or derived elements thereof with respect to the placement information.

According to some embodiments, the signal source specific analysis comprises signal separation or source separation, wherein the signals and the respective placement information may be combined to create at least one signal of a respective source which comprises fewer signal components of other signal sources.

According to some embodiments, the signal source specific analysis comprises noise suppression, wherein the signals and the respective placement information are combined to create a signal with a lower noise level.

According to some embodiments, the signal source specific analysis comprises beamforming, wherein the signals and the respective placement information are combined to create a directional beam of higher gain in a particular direction while reducing interference and noise from other directions.

According to some embodiments, the signal source specific analysis comprises location specific diagnosis, wherein the signals and the respective placement information are used to generate a diagnosis regarding a specific location within the body part, which might also be one of the auscultatory sources of one of the signals.

According to some embodiments, the signal source specific analysis comprises differential diagnostic, wherein the signals and the respective placement information are used to generate a diagnosis from differential information such as signal parts, features, model parameters or the like of at least two signals.

As used herein, the term "source" refers to a body part whose activity is causal to a corresponding signal measurable by the corresponding sensor, such as a heart valve; a joint or the lungs being an acoustic source of an acoustic signal measured by the PG sensors or the heart being the electric source of an electric signal measured by an ECG sensor.

As used herein, the term "diagnosis" refers to the identification of a disease, condition or fitness of a body part or its function.

According to some embodiments, the conjoint analysis is based on additional inputs. According to some embodiments, the additional inputs include information related to the subject, such as age, gender, height, weight, clinical state, clinical history, medication, comorbidities, genetic information, or other demographic information. According to some embodiments, the additional inputs include signals obtained from additional sensors other than the PG sensors or BIO sensor. According to some embodiments, the additional inputs are provided from an external device or sensor, and/or by a user of the device, for example, via input means facilitated by the device.

As used herein, a computational model refers to a program executed/executable on a computer, configured to receive certain inputs, apply certain computations or algorithms, and generate at least one output. A computational model can be a machine learning algorithm, a filtering operation, a derivation model, a convolution model, or the like.

As used herein, the term "body unit" refers to a part of the signal processing unit, which is placed on the body of the subject. It may comprise an integrated computation resource of the signal processing unit or a separate computation resource may be comprised instead. For communicating (e.g., with a separate computation resource) the body unit may include a body unit communication unit. Preferably, the signal processing unit comprises a body unit and at least one computation resource integrated in a body unit of the signal processing unit.

As used herein, the term "computation resource" refers to a component of the signal processing unit on which the signal source specific analysis according to the invention can actually be performed. The computation resource may be a dedicated computation resource of the body unit or in some embodiments this can be a separate computation resource. The separate computation resource may be integrated in a dedicated mobile unit of a device according to the present invention or integrated in an external device such as a smartphone, tablet, computer or other wearable devices. In some embodiments, the body unit may include an internal computation resource in combination with a separate computation resource. The internal and the separate computation resource may be used alternatively or the computation is cooperatively running on both computation resources.

In one embodiment of the invention, the signal processing unit comprises a body unit and at least one separate computation resource, and the body unit further comprises a body unit communication unit configured to communicate data with a computation resource communication unit of the computation resource. In another embodiment, the computation resource may be integrated in the body unit. The computation resource may compute steps such as the computation of the signal source specific analysis in parallel or distinct steps individually. According to some embodiment, a computation step is performed on the signal processing unit of the body unit, necessary data is transmitted via the different communication units to the separated computation resource, where another computation step is performed, some data is transmitted back to the signal processing unit of the body unit for the next computation step. Multiple separated computation resources could communicate with each other or only with the body unit. Advantageously, such configurations minimize energy consumption on the body unit or the size of the body unit or the need for redundant or special computational equipment, all of which would be beneficial on a wearable device.

In one embodiment of the invention, the computation resource is configured to fully or partially conjointly perform the signal source specific analysis by combining the information of the PG signals with a respective placement information where the PG sensors are arranged on the body part and the information of the BIO signal.

In one embodiment of the invention, all or parts of the PG sensors and BIO sensor are connected wirelessly to the signal processing unit (e.g., a body unit of the signal processing unit), wherein any of said sensors further comprise a sensor communication unit configured to obtain any of the respective PG signals from the PG sensors or BIO signal from the BIO sensor, and to transmit said signals to the signal processing unit. In particular, said signals may be transmitted to a body unit communication unit on the body unit which is configured to receive said signals. Advantageously, such a configuration can also be used to measure auscultation areas that are far apart or require varying distance, such as the two knee joints. According to some aspects, one or more of the sensor signals transmitted to the device are processed before transmission. The processing of the signal can include one or more of analog to digital conversions, digital-to-analog conversions, noise reduction, high-frequency filtration, low-frequency filtration, band filtration, modulation, adjusting an amplitude/gain of the signal, encryption, compression, dimension reduction, adaptive filtering, feature extraction, a machine learning model inference, or the like.

As used herein, the term "placement information" refers to the specific locations of the auscultatory areas on the subject's body where the PG sensors are arranged to measure or monitor the PG signals. In one embodiment of the invention, this information may be given in the form of the name of the signal indicating the specific location of the auscultatory area, such as a mitral valve PG sensor which would indicate a position on the auscultatory area of the mitral valve of the heart, or a left lung PG sensor which would indicate a position on the auscultatory area of the left lung. The specific location of the auscultatory area must be specific in terms of relative positioning to the other PG sensors, as an example two PG sensor with the placement information "left lung" and "right lung" would be sufficient to indicate their specific relative position, but two PG sensors with the placement information "left lung" and "left lung" would not be sufficiently specific.

According to some aspects, the device has to be placed directly on the subjects body and to hold the device in desired positions, self-adhesive sensor interfaces such as electrodes or optodes can be advantageously used to optimize space, in addition to or instead of the adhesion of the sensor interface, the device can be positioned in place using a belt, lanyard, suction cup (with or without active vacuum), can be handheld in position, or be textile integrated, the device can include an adhesive member surrounding the sensor interface and/or placed in spaces between sensor interfaces, or the device can be covered by plaster or other adhesive such that the device is at least partially or fully covered by the plaster, by applying a separate glue between device and skin, by using an additional double-sided adhesive, by placing an interface member on the skin which stays on the skin while the device can be attached to and removed from that interface, or the like.

Another aspect of the invention relates to a method for conjointly performing signal source specific analysis in a device according to the invention, comprising combining the information of PG signals with their respective placement information and the information of a BIO signal.

Certain embodiments of the present disclosure may include some, all, or none of the above advantages. One or more technical advantages may be readily apparent to those skilled in the art from the figures, descriptions, and claims included herein. Moreover, while specific advantages have been enumerated above, various embodiments may include all, some, or none of the enumerated advantages. In addition to the exemplary aspects and embodiments described above, further aspects and embodiments will become apparent by reference to the figures and by the study of the following detailed descriptions.

All embodiments described in this specification may be advantageously combined with one another to the extent that their respective features are compatible. In particular, the expressions "according to an embodiment", "in an embodiment", "an embodiment of the invention provides" etc. mean that the respective features may or may not be part of specific embodiments of the present invention.

### Brief Description of the Drawings

Examples illustrative of embodiments are described below with reference to the figures attached hereto. In the figures, identical structures, elements, or parts that appear in more than one figure are generally labeled with the same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.
Fig. 1 schematically illustrates the placement of the sensors for heart auscultation in combination with EG sensors, according to some embodiments;
Fig. 2 schematically illustrates the flow chart of a signal source specific analysis for heart auscultation in combination with EG signals, according to some embodiments;
Fig. 3 schematically illustrates a portion of a single PG signal and a single EG signal, according to some embodiments;
Fig. 4 schematically illustrates the placement of the sensors for heart auscultation in combination with IG sensors, according to some embodiments;
Fig. 5 schematically illustrates the flow chart of a signal source specific analysis for heart auscultation in combination with an IG signal, according to some embodiments;
Fig. 6 schematically illustrates a portion of a single PG signal and the filtered PG signal after signal separation, according to some embodiments;
Fig. 7 schematically illustrates the placement of the sensors for lung auscultation in combination with IG sensors, according to some embodiments;
Fig. 8 schematically illustrates the flow chart of a signal source specific analysis for lung auscultation in combination with an IG signal, according to some embodiments;
Fig. 9 schematically illustrates the placement of the sensors for knee joint auscultation in combination with IG sensors, according to some embodiments;
Fig. 10 schematically illustrates the flow chart of a signal source specific analysis for knee joint auscultation in combination with an IG signal, according to some embodiments; and
Fig. 11 schematically illustrates the placement of the sensors for knee joint auscultation in combination with IG sensors and the setup of the device with a signal processing unit comprising a body unit with a body unit communication unit, a separated computation resource with a computation resource communication unit and a sensor communication unit, according to some embodiments.

### Detailed Description of the Drawings

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

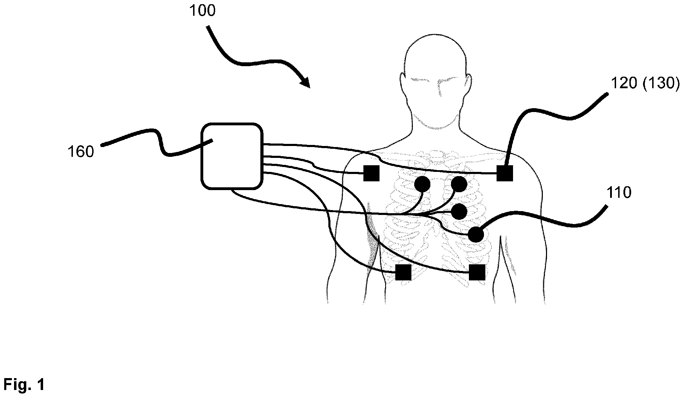

Reference is now made to Fig. 1, which schematically illustrates the placement of the sensors for heart auscultation in combination with EG sensors 130, according to some embodiments. The sensors of the device 100 are shown as four PG sensors 110 and four EG sensors 130 as BIO sensors 120, wherein the PG sensors 110 cover the four distinct auscultation areas of the heart over the aortic, pulmonic, tricuspid, and mitral heart valves. In this measurement setup, the four EG sensors 130 are arranged as a 3-channel ECG with another electrode as a reference node. However, it should be noted that other measurement configurations are also possible, both for the placement of the EG sensors 130 and the PG sensors 110. The device 100 may be connected with the sensors in a wired manner, as in this example, or in a wireless manner, as will be explained in more detail in a following example. The sensors may also be integrated into or attached to the housing of the device 100, and thereby used as a wearable device 100. In the following sensor placement illustrations, the device 100, its connections to the sensors, and its signal processing unit 160 are not shown for simplicity.

The signal processing unit 160 of the device 100 receives from the sensors four PG signals 115 and three electrical potential signals from which three differential voltage signals can be obtained, referred to as EG signals 135, which serve as BIO signals 125. As in all the following examples, these signals are either measured synchronously or synchronized by using their time stamps. All signals can be filtered appropriately, e.g., the PG signals 115 can be filtered with a bandpass filter between 5 Hz and 250 Hz and the EG signals 135 with a bandpass filter between 0.5 Hz and 100 Hz, although other filter types and techniques such as baseline removal, transformation based filters, noise reduction filters, or the like are equally suitable.

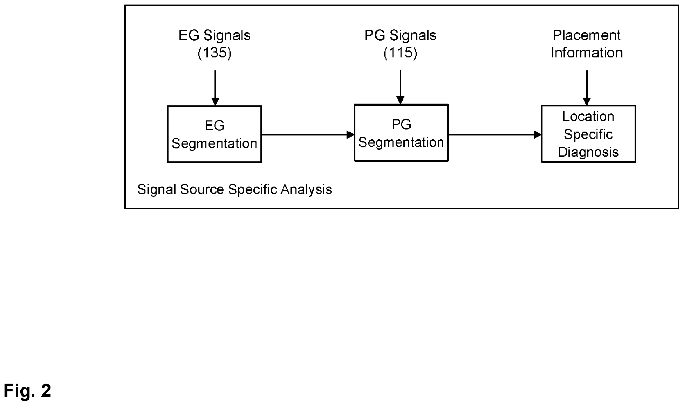

Reference is now made to Fig. 2, which schematically illustrates the flow chart of a signal source specific analysis for heart auscultation in combination with EG signals 135, according to some embodiments. The signal source specific analysis is comprised of the steps EG segmentation, which takes at least one EG signal 135 as an input and produces a segmentation of the EG signal 135, followed by a PG segmentation, which takes the prior EG segmentation with or without the EG signal 135 and the PG signals 115 as input and produces segmentations of the PG signals 115, and lastly, a location specific diagnosis, which takes the prior EG segmentation, PG segmentation, potentially the raw signals or features thereof and the placement information as input and provides a diagnosis for specific sites of the heart, such as different heart valves.

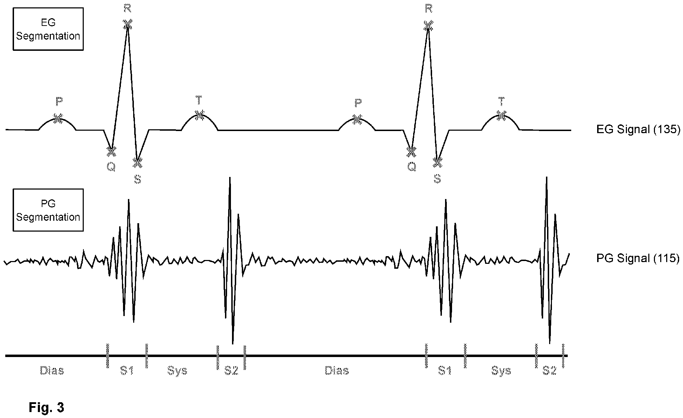

Reference is now made to Fig. 3, which schematically illustrates a portion of a single PG signal 115 and a single EG signal 135 received from the sensor arrangement explained before, according to some embodiments. On Fig. 3 not only the EG signal 135 and PG signal 115 is shown but also the results of the EG segmentation and PG segmentation.

According to one embodiment, a EG cardiac cycle segmentation is performed from at least one of the EG signals 135, whereby the cardiac cycles and the timings of the PQRST complex are detected. Using the determined time points and segments of the EG signals 135, the PG signals 115 are also segmented into cardiac cycles and the segments of S1, S2, diastole and systole for each cardiac cycle are determined. Different ways of processing of EG and PG cardiac signals and the associated techniques and processing steps may occur to those skilled in the art, so that adjustments could be made, such as detecting possible S3 and S4 sounds in the PG signal 115 or detecting the J point or U wave in the EG signal 135. The following location specific diagnosis takes the PG signal 115, its corresponding segments from the PG segmentation (S1, S2, systole and diastole), the EG signal 135 and its corresponding segments from the EG segmentation (P, Q, R, S and T) as input and produces at least one diagnosis specific to one of the sources, e.g., the mitral valve. To achieve this, features of specific segmented parts of signals from specific placements are calculated using various feature generating techniques based on amplitudes, times, frequencies, properties of transforms, statistical properties, or the like known to those skilled in the art. As an example, the signal power of the S1 segment in the PG signal 115 from the mitral auscultation site is divided by signal power of the S2 segment in the PG signal 115 from the aortic auscultation site, which is used with a threshold value to provide a diagnosis for the mitral valve.

It is noted that many different features of similar kind are equally feasible, where in the signal source specific analysis information from the PG signals 115 and the corresponding placement information of the PG sensors 110 is combined with the information form the BIO signal 125 - in this case the EG signal 135. Here, one of the EG signals 135 is used to segment the PG signals 115, which is then used in combination with the placement information to create a location specific diagnosis for the mitral valve using the signal from the PG signal 115 from the mitral auscultation site and the aortic auscultation site.

Alternatively, the stated feature and various other features might be processed in a machine learning algorithm, which was trained on data from subjects with known cardiac diseases to produce cardiac diagnoses. It is noted here that various techniques and features could be used to produce similar results, which can be easily adapted from the present disclosure for the expert.

Advantageously, by using the combined information of the PG signal 115, the corresponding placement information of the PG sensors 110 and the BIO signal 125, which is conjointly analyzed in a signal source specific analysis, a degree of diagnostic accuracy can be achieved, which performs superior to human auscultation, sequential auscultation and other auscultation techniques which differ from the embodiments of the presented invention.

Alternatively, in this example instead of using an EG sensor 130 as BIO sensor 120, a PPG sensor 150 could be used as BIO sensor 120 to perform an equally feasible PPG segmentation, which would enable a similar result in the EG segmentation.

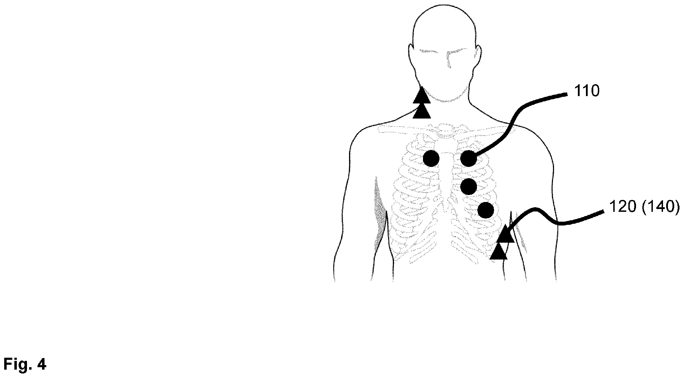

Reference is now made to Fig. 4, which schematically illustrates the placement of the sensors for heart auscultation in combination with IG sensors 140, according to some embodiments. The sensors of the device 100 are shown as four PG sensors 110 and four IG sensors 140 as BIO sensors 120, wherein the PG sensors 110 cover the four distinct auscultation areas of the heart over the aortic, pulmonic, tricuspid, and mitral heart valves. In this measurement setup, the four IG sensors 140 are arranged to either inject current (DC or AC, depending on the application) and measure voltage or vice versa in pairs through the thorax to estimate hemodynamic pressure, flow, vascular resistance, or the like in the heart. However, it should be noted that other measurement configurations are also possible, both for the amount and placement of the IG sensors 140 and the PG sensors 110. For example, a similar measurement setup can be performed with two IG sensors 140 that simultaneously induce current and measure voltage, although in most applications this is less accurate due to the voltage-dependent resistance of the skin, in some cases the skin impedance might be desirable such as in the galvanic skin response. Alternatively, more IG sensors 140 might be used to perform impedance based imaging.

The signal processing unit 160 of the device 100 receives from the sensors two PG signals 115 and one impedance signal, referred to as IG signal 145, which serves as BIO signal 125. All signals can be filtered appropriately using various filtering techniques such as lowpass, highpass, notch or bandpass filters, baseline removal filters, transformation-based filters, noise reduction filters, or the like.

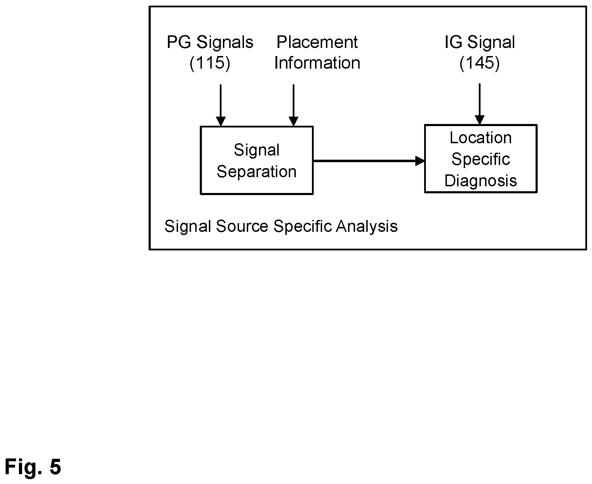

Reference is now made to Fig. 5, which schematically illustrates the flow chart of a signal source specific analysis for heart auscultation in combination with an IG signal 145, according to some embodiments. The signal source specific analysis comprises of the steps signal separation, which takes the PG signals 115 and the corresponding placement information of the PG sensors 110 as an input and produces at least one source separated PG signal 115, followed by a source diagnosis, which takes the prior separated PG signals 115 and the IG signal 145 as input and produces a diagnosis for the specific signal sources.

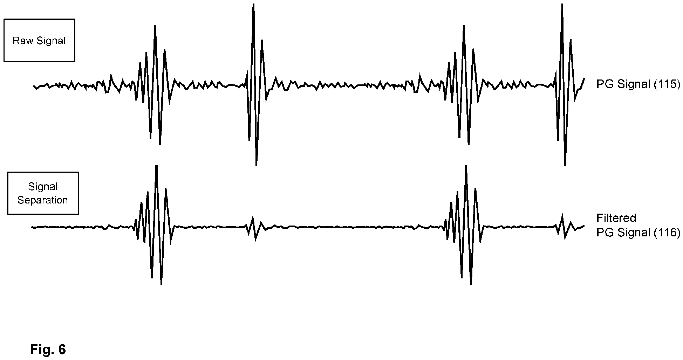

Reference is now made to Fig. 6, which schematically illustrates a portion of a single PG signal 115 and the filtered PG signal 116 after signal separation, according to some embodiments. The PG signals 115 and the corresponding placement information of the PG sensors 110 are used to separate the signals by using the knowledge of the auscultation sites and its corresponding sources. Exemplarily, the signal separation step aims to separate the first heart sound (S1), using the knowledge that the S1 sound arises mainly from mitral valve closure, but may also contain components of tricuspid valve closure, while the second heart sound (S2) arises mainly from the aortic valve closure with components of the pulmonal valve closure. Thus, with the four PG signals 115 and the corresponding placement information of the PG sensors 110, it is possible to separate or amplify the S1 sound from the other sound signals using standard signal processing steps known to those skilled in the art, such as template matching, PCA, ICA, empirical mode decomposition, DTW, or even machine learning models to produce a filtered PG signal 116. In a similar way, other filtered PG signals 116 can be calculated, for example, for the other heart valves.

With the filtered PG signal 116 and the information from an additional IG signal 145, it is possible to produce a location specific diagnosis, in this case for the mitral valve, wherein the filtered PG signals 116 and the separated S1 sound is analyzed conjointly with the IG signal 145, to detect a stiffness or leakage of the mitral heart valve through fluttering or side noise in the PG signals 115 or differences and asynchrony between S1 sound and IG signal 145 flow or pressure estimations.

It is reiterated that various techniques could be used to achieve similar results that can be easily adapted from the present disclosure for those skilled in the art, such as the use of machine learning algorithms trained on subject data with known cardiac diseases.

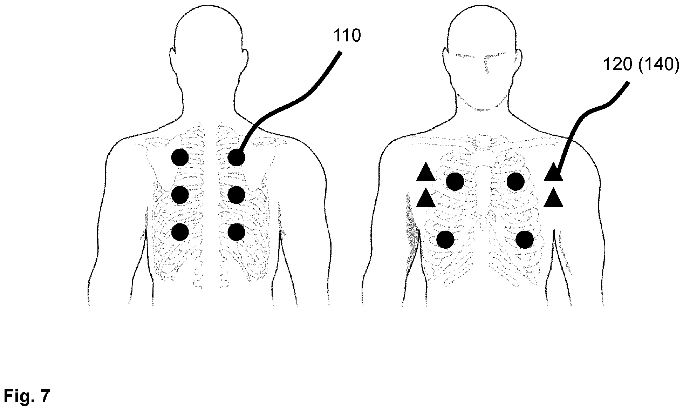

Reference is now made to Fig. 7, which schematically illustrates the placement of the sensors for lung auscultation in combination with IG sensors 140, according to some embodiments. The sensors of the device 100 are shown as ten PG sensors 110 and four IG sensors 140 as BIO sensors 120, with the PG sensors 110 covering specific auscultation areas of the lung over the upper and lower lobes of the right and left lungs from the front and the upper, middle and lower lobes of the right and left lungs from the back. In this measurement setup, the four IG sensors 140 are arranged to induce current (AC with varying frequencies) and measure voltage in pairs through the thorax over different frequencies for lung impedance spectroscopy. However, it should be noted that other measurement configurations are also possible, both for the placement of the IG sensors 140 and the PG sensors 110. For example, the impedance sensors could be arranged to simultaneously induce current and measure voltage, or could be arranged in a completely different manner, such as for impedance pneumography to estimate lung volume or with more electrodes for impedance tomography to image tissue.

The signal processing unit 160 of the device 100 receives from the sensors ten PG signals 115 and one impedance signal, referred to as IG signal 145, which serves as BIO signal 125. Filtering can be applied to all signals.

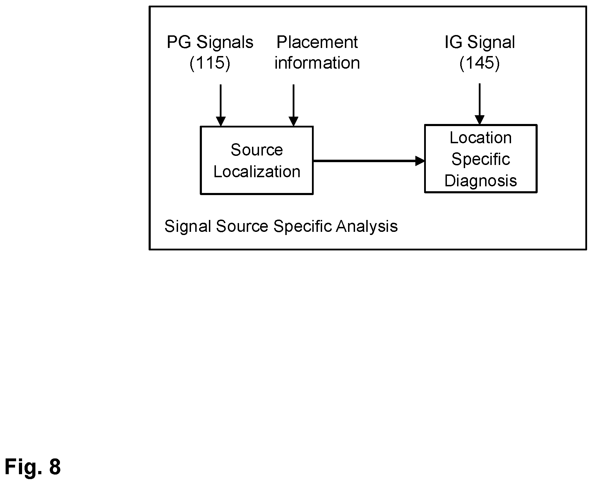

Reference is now made to Fig. 8, which schematically illustrates the flow chart of a signal source specific analysis for lung auscultation in combination with an IG signal 145, according to some embodiments. The signal source specific analysis is comprised of the steps source localization, which takes the PG signals 115 and the corresponding placement information of the PG sensors 110 as an input and produces an estimation of a possible localization of liquid in the lung, followed by a location specific diagnosis, which takes the result of the prior source localization and the IG signal 145 as input and produces a diagnosis for the amount and position of liquid or inflammation in the lung.

The PG signals 115 and the respective placement information of the PG sensors 110 are analyzed conjointly using differential analysis to detect crackles and wheezes in the lung breath sounds, which are different from breath sounds of other lung sections, because in most cases inflammation and/or fluid in the lung occurs only locally and not in the entire lung. From this, the inflamed lung area or area with fluid will be estimated first, and then lung impedance spectroscopy analysis will be performed to estimate the amount of inflamed tissue or fluid in the lung, exemplary by using the impedance spectroscopy data to fit a cole-cole model to estimate the fluid content. Together this information can be used to diagnose the area and degree of inflammation/fluid in the lung.

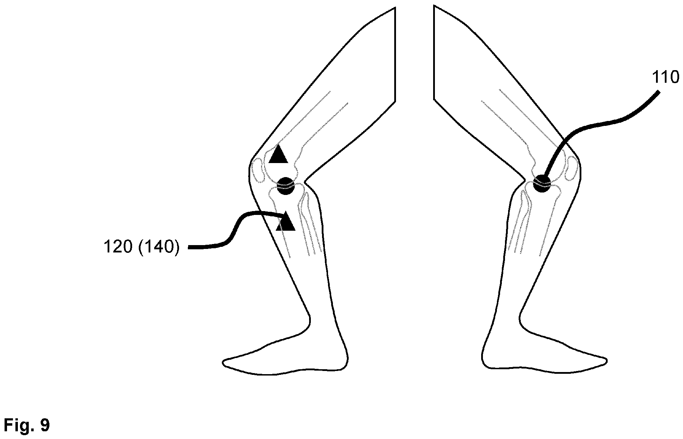

Reference is now made to Fig. 9, which schematically illustrates the placement of the sensors for knee joint auscultation in combination with IG sensors 140, according to some embodiments. The sensors of the device 100 are shown as two PG sensors 110 and two IG sensors 140 as BIO sensors 120, wherein the PG sensors 110 cover an auscultation area of each knee. In this measurement setup, the two IG sensors 140 are arranged to simultaneously induce current (AC with varying frequency) and measure voltage through a single knee over different frequencies for joint impedance spectroscopy.

The signal processing unit 160 of the device 100 receives from the sensors two PG signals 115 and one impedance signal, referred to as IG signal 145, which serves as BIO signal 125. Filtering might be applied to all signals.

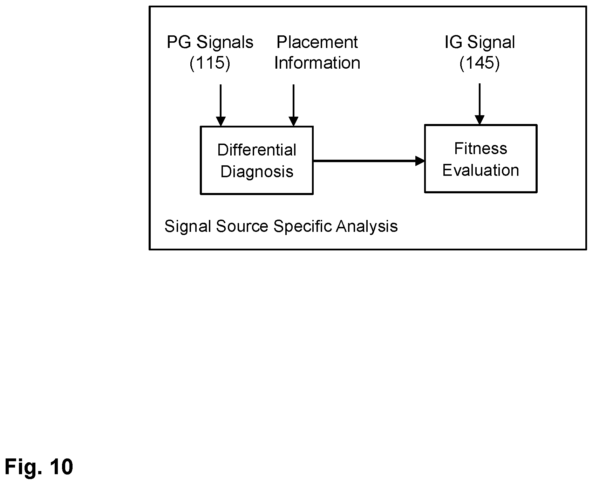

Reference is now made to Fig. 10, which schematically illustrates the flow chart of a signal source specific analysis for knee joint auscultation in combination with an IG signal 145, according to some embodiments. The signal source specific analysis comprises of the steps differential diagnostics, which takes the PG signals 115 and the corresponding placement information of the PG sensors 110 as an input and produces a location specific diagnosis of the varying fitness between the two auscultated joints for each joint, followed by a signal source specific fitness evaluation, which takes the result of the prior differential diagnostics and the IG signal 145 as input and produces a fitness estimation of the knee with the IG sensors 140 attached.

The PG signals 115 are differentially analyzed to detect joint sounds during synchronous movements of both knee joints and this is combined with the respective placement information of the PG sensors 110 to establish a differential diagnosis of the knees which is location specific. This information is combined with joint impedance spectroscopy, which further analyzes the tissue for potential inflammation or liquid in one knee to provide an assessment of the fitness of that knee compared to the other knee.

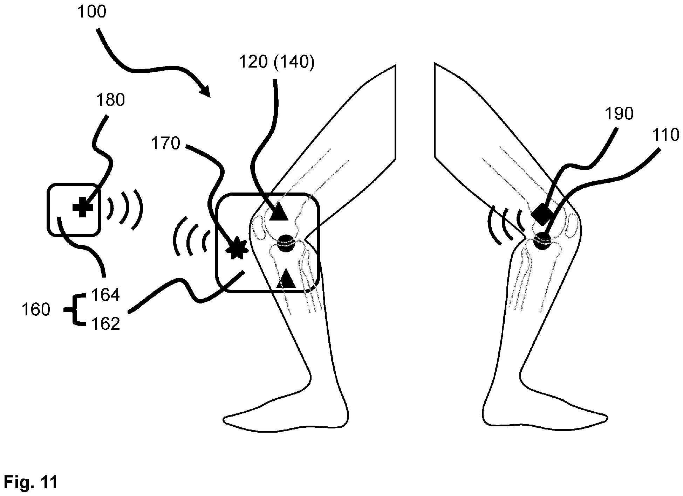

Reference is now made to Fig. 11, which schematically illustrates the placement of the sensors for knee joint auscultation in combination with IG sensors 140 and the setup of the device 100 with a signal processing unit 160 comprising a body unit 162 with a body unit communication unit 170, a separated computation resource 164 with a computation resource communication unit 180 and a sensor communication unit 190, according to some embodiments. With the sensor setup and the computation steps as previously explained, the device 100 comprises a signal processing unit 160 including a body unit 162 on the left knee (from the viewer's perspective) and a body unit communication unit 170 and which is connected to the PG sensor 110 and the IG sensors 140 arranged on the left knee. The body unit 162 is placed as wearable device 100 on the left knee. The PG sensor 110 on the right knee comprises a sensor communication unit 190, which is connected wirelessly to the body unit communication unit 170 of the body unit 162. The sensor communication unit 190 obtains the PG signal 115 from the PG sensor 110 on the right knee, possibly preprocesses the PG signal 115 and wirelessly transmits the PG signal 115 to the body unit communication unit 170 of the body unit 162. After the body unit 162 has obtained and possibly preprocessed all signals wired and wirelessly, it transmits the data with its body unit communication unit 170 to a computation resource communication unit 180 of a separated computation resource 164, which is locally separated from the body unit 162. The separated computation resource 164 might perform some or all computation steps of the signal source specific analysis.

Alternatively, there may be a wired connection from all sensors to the body unit 162 but a wireless connection between the body unit 162 and the separated computation resource 164, there may be no separate computation resource 164, there may be multiple separate computation resources 164, there may be multiple wirelessly connected sensors comprising of either one or multiple sensor communication units 190, there may be a wireless connection from the sensor directly to the separate computation resource 164, there may be multiple data transfers between multiple separate computation resources 164 or back to the body unit 162, there may be a separated computation resource 164 which acts as display or control device 100, or the like.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" or "comprising," when used in this specification, specify the presence of stated features, integers, steps, operations, elements, or components, but do not preclude or rule out the presence or addition of one or more other features, integers, steps, operations, elements, components, or groups thereof.

While a number of exemplary aspects and embodiments have been discussed above, those of skill in the art will recognize certain modifications, additions, and sub-combinations thereof. It is therefore intended that the following appended claims and claims hereafter introduced be interpreted to include all such modifications, additions, and sub-combinations as are within their scope.

### Reference Signs

- 100: Device
- 110: PG sensor
- 115: PG signal
- 116: Filtered PG signal
- 120: BIO sensor
- 125: BIO signal
- 130: EG sensor
- 135: EG signal
- 140: IG sensor
- 145: IG signal
- 150: PPG sensor
- 155: PPG signal
- 160: Signal processing unit
- 162: Body unit
- 164: Computation resource
- 170: Body unit communication unit
- 180: Computation resource communication unit
- 190: Sensor communication unit

## Claims

1. A multi-sensor auscultation device (100) for measuring and/or monitoring a body part of a subject, the device (100) comprising:
- a plurality of surface vibration sensitive phono-gram sensors, PG sensors (110), each configured to measure an acoustic activity of the body part and each to provide a PG signal (115) indicative of the acoustic activity of the body part, wherein the PG sensors (110) are adapted to be arranged on the body part to cover different distinct auscultation areas of said body part;
- at least one other biomedical sensor, BIO sensor (120), configured to measure in dependence of the sensor type a corresponding activity of the body part and to provide at least one BIO signal (125) indicative of the corresponding activity of the body part;
- a signal processing unit (160), configured to
- obtain the signals (115, 125) from the sensors (110, 120), **characterised in that** the signal processing unit is configured to conjointly perform a signal source specific analysis by combining the information of the PG signals (115) with a respective placement information where the PG sensors (110) are arranged on the body part and the information of the BIO signal (125).

2. The device (100) of claim 1, wherein the device (100) comprises as BIO sensors (120) at least one of:
- a surface electrical potential differentials sensor, especially an electro-gram sensor, EG sensor (130), configured to measure an electrical activity of the body part and to provide at least one EG signal (135) indicative of the electrical activity of the body part;
- an impedance sensor, especially an impedance-gram sensor, IG sensor (140), configured to measure an electrical impedance of the body part and to provide an IG signal (145) indicative of the electrical impedance of the body part; and
- a photoplethysmography sensor, PPG sensor (150), configured to measure changes in blood volume within tissues of the body part and to provide a PPG signal (155) indicative of pulsatile variations in blood volume, such as the arterial blood flow, heart rate, blood oxygen saturation, or the overall perfusion status of the body part.

3. The device (100) of claim 1 or 2, wherein the signal processing unit (160) comprises a body unit (162) and at least one separate computation resource (164), and the body unit (162) comprises a body unit communication unit (170) configured to communicate with a computation resource communication unit (180) of the computation resource (164).

4. The device (100) of claim 3, wherein the computation resource (164) is configured to fully or partially conjointly perform the signal source specific analysis by combining the information of the PG signals (115) with a respective placement information where the PG sensors (110) are arranged on the body part and the information of the BIO signal (125).

5. The device (100) of any one of the preceding claims, wherein all or parts of the PG sensors (110) and BIO sensor (120) are connected wirelessly to the signal processing unit (160); wherein any of said sensors (110, 120) further comprise a sensor communication unit (190) configured to obtain any of the respective signals (115, 125) from the sensors (110, 120) and to transmit said signals (115, 125) to the signal processing unit (160).

6. The device (100) of any one of the preceding claims, wherein the device (100) is a wearable device.

7. The device (100) of any one of the preceding claims, wherein the signal source specific analysis comprises location specific analysis that provides a location specific analysis of a specific site within the body part.

8. The device (100) of any one of the preceding claims, wherein the signal source specific analysis comprises signal separation that provides at least one signal (115, 125) which comprises fewer signal components of other signal sources.

9. The device (100) of any one of the preceding claims, wherein the signal source specific analysis comprises noise suppression that provides at least one signal (115, 125) with a stronger signal component of at least one signal source.

10. The device (100) of any one of the preceding claims, wherein the signal source specific analysis comprises differential diagnosis that provides a diagnosis with respect to differential information of multiple signals (115, 125).

11. The device (100) of any one of the preceding claims, wherein the body part is a heart of the subject, and the multiple PG-sensors (110) are arranged, that at least two of the four major auscultatory areas of the heart are covered.

12. The device (100) of claim 10, wherein there are at least four multiple PG-sensors (110) arranged to cover all four major auscultatory areas of the heart.

13. The device (100) of any one of claims 1 to 10, wherein the body part is the lung of the subject.

14. The device (100) of any one of claims 1 to 10, wherein the body part is a joint of the subject.

15. A method for conjointly performing signal source specific analysis in a device of any one of the preceding claims, comprising combining the information of PG signals (115) with their respective placement information and the information of a BIO signal (125).

## Patentansprüche

1. Auskultationsvorrichtung (100) mit mehreren Sensoren zum Messen und/oder Überwachen eines Körperteils eines Objekts, wobei die Vorrichtung (100) umfasst:
- eine Vielzahl von oberflächenvibrationsempfindlichen Phonogramm-Sensoren, PG-Sensoren (110), die jeweils konfiguriert sind, um eine akustische Aktivität des Körperteils zu messen und jeweils ein PG-Signal (115) zu liefern, das die akustische Aktivität des Körperteils anzeigt, wobei die PG-Sensoren (110) angepasst sind, um auf dem Körperteil angeordnet zu werden, um verschiedene unterschiedliche Auskultationsbereiche des besagten Körperteils abzudecken;
- mindestens einen weiteren biomedizinischen Sensor, BIO-Sensor (120), der konfiguriert ist, um in Abhängigkeit von dem Sensortyp eine entsprechende Aktivität des Körperteils zu messen und mindestens ein BIO-Signal (125) zu liefern, das die entsprechende Aktivität des Körperteils anzeigt;
- eine Signalverarbeitungseinheit (160), die konfiguriert ist, um
- die Signale (115, 125) von den Sensoren (110, 120) zu erhalten,
**dadurch gekennzeichnet, dass** die Signalverarbeitungseinheit konfiguriert ist, um
- gemeinsam eine signalquellenspezifische Analyse durch Kombinieren der Information der PG-Signale (115) mit einer jeweiligen Platzierungsinformation, wo die PG-Sensoren (110) am Körperteil angeordnet sind, und der Information des BIO-Signals (125) durchzuführen.

2. Vorrichtung (100) nach Anspruch 1, wobei die Vorrichtung (100) als BIO-Sensoren (120) mindestens einen von Folgenden umfasst:
- einen Sensor für elektrische Potentialdifferenzen an der Oberfläche, insbesondere einen Elektrogrammsensor, EG-Sensor (130), der konfiguriert ist, um eine elektrische Aktivität des Körperteils zu messen und mindestens ein EG-Signal (135) zu liefern, das die elektrische Aktivität des Körperteils anzeigt;
- einen Impedanzsensor, insbesondere einen Impedanzgrammsensor, IG-Sensor (140), der konfiguriert ist, um eine elektrische Impedanz des Körperteils zu messen und ein IG-Signal (145) zu liefern, das die elektrische Impedanz des Körperteils anzeigt; und
- einen Photoplethysmographie-Sensor, PPG-Sensor (150), der konfiguriert ist, um Änderungen des Blutvolumens in Geweben des Körperteils zu messen und ein PPG-Signal (155) zu liefern, das pulsierende Änderungen des Blutvolumens anzeigt, wie den arteriellen Blutfluss, die Herzfrequenz, die Sauerstoffsättigung des Blutes oder den Gesamtperfusionsstatus des Körperteils.

3. Vorrichtung (100) nach Anspruch 1 oder 2, wobei die Signalverarbeitungseinheit (160) eine Körpereinheit (162) und mindestens eine separate Rechenressource (164) umfasst, und die Körpereinheit (162) eine Körpereinheit-Kommunikationseinheit (170) umfasst, die konfiguriert ist, um mit einer Rechenressourcen-Kommunikationseinheit (180) der Rechenressource (164) zu kommunizieren.

4. Vorrichtung (100) nach Anspruch 3, wobei die Rechenvorrichtung (164) konfiguriert ist, um die signalquellenspezifische Analyse ganz oder teilweise gemeinsam durchzuführen, indem sie die Information der PG-Signale (115) mit einer entsprechenden Platzierungsinformation, wo die PG-Sensoren (110) am Körperteil angeordnet sind, und der Information des BIO-Signals (125) kombiniert.

5. Vorrichtung (100) nach einem der vorhergehenden Ansprüche, wobei alle oder Teile der PG-Sensoren (110) und des BIO-Sensors (120) drahtlos mit der Signalverarbeitungseinheit (160) verbunden sind; wobei jeder der besagten Sensoren (110, 120) ferner eine Sensor-Kommunikator-Einheit (190) umfasst, die konfiguriert ist, um jedes der jeweiligen Signale (115, 125) von den Sensoren (110, 120) zu erhalten und die besagten Signale (115, 125) an die Signalverarbeitungseinheit (160) zu übertragen.

6. Vorrichtung (100) nach einem der vorhergehenden Ansprüche, wobei die Vorrichtung (100) eine tragbare Vorrichtung ist.

7. Vorrichtung (100) nach einem der vorhergehenden Ansprüche, wobei die signalquellenspezifische Analyse eine positionsspezifische Analyse umfasst, die eine positionsspezifische Analyse eines bestimmten Ortes innerhalb des Körperteils liefert.

8. Vorrichtung (100) nach einem der vorhergehenden Ansprüche, wobei die signalquellenspezifische Analyse eine Signaltrennung umfasst, die mindestens ein Signal (115, 125) liefert, das weniger Signalkomponenten anderer Signalquellen umfasst.

9. Vorrichtung (100) nach einem der vorhergehenden Ansprüche, wobei die signalquellenspezifische Analyse eine Rauschunterdrückung umfasst, die mindestens ein Signal (115, 125) mit einer stärkeren Signalkomponente von mindestens einer Signalquelle liefert.

10. Vorrichtung (100) nach einem der vorhergehenden Ansprüche, wobei die signalquellenspezifische Analyse eine Differenzialdiagnose umfasst, die eine Diagnose in Bezug auf Differenzialinformationen mehrerer Signale (115, 125) liefert.

11. Vorrichtung (100) nach einem der vorhergehenden Ansprüche, wobei es sich bei dem Körperteil um das Herz des Objekts handelt und die mehreren PG-Sensoren (110) so angeordnet sind, dass mindestens zwei der vier Hauptauskultationsbereiche des Herzens abgedeckt sind.

12. Vorrichtung (100) nach Anspruch 10, wobei mindestens vier mehrere PG-Sensoren (110) vorhanden sind, die so angeordnet sind, dass sie alle vier Hauptauskultationsbereiche des Herzens abdecken.

13. Vorrichtung (100) nach einem der Ansprüche 1 bis 10, wobei der Körperteil die Lunge des Objekts ist.

14. Vorrichtung (100) nach einem der Ansprüche 1 bis 10, wobei der Körperteil ein Gelenk des Objekts ist.

15. Verfahren zum gemeinsamen Durchführen einer signalquellenspezifischen Analyse in einer Vorrichtung nach einem der vorhergehenden Ansprüche, umfassend das Kombinieren der Information von PG-Signalen (115) mit ihrer jeweiligen Platzierungsinformation und der Information eines BIO-Signals (125).

## Revendications

1. Dispositif d'auscultation à capteurs multiples (100) pour mesurer et/ou surveiller une partie du corps d'un objet, le dispositif (100) comprenant :
- une pluralité de capteurs de phono-grammes sensibles aux vibrations de surface, capteurs PG (110), chacun étant configuré pour mesurer une activité acoustique de la partie du corps et chacun pour fournir un signal PG (115) indiquant l'activité acoustique de la partie du corps, dans lequel les capteurs PG (110) sont adaptés pour être disposés sur la partie du corps afin de couvrir différentes zones d'auscultation distinctes de ladite partie du corps ;
- au moins un autre capteur biomédical, capteur BIO (120), configuré pour mesurer, en fonction du type de capteur, une activité correspondante de la partie du corps et pour fournir au moins un signal BIO (125) indiquant l'activité correspondante de la partie du corps ;
- une unité de traitement de signal (160), configurée pour
- obtenir les signaux (115, 125) depuis les capteurs (110, 120),
**caractérisé en ce que** l'unité de traitement de signal est configurée pour
- réaliser conjointement une analyse spécifique à la source de signal en combinant les informations des signaux PG (115) avec une information de placement respectif où les capteurs PG (110) sont disposés sur la partie du corps et les informations du signal BIO (125).

2. Dispositif (100) de la revendication 1, dans lequel le dispositif (100) comprend en tant que capteurs BIO (120) au moins un parmi :
- un capteur de différentiel de potentiel électrique de surface, notamment un capteur d'électro-grammes, capteur EG (130), configuré pour mesurer une activité électrique de la partie du corps et pour fournir au moins un signal EG (135) indiquant l'activité électrique de la partie du corps ;
- un capteur d'impédance, en particulier un capteur d'impédance-gramme, capteur IG (140), configuré pour mesurer une impédance électrique de la partie du corps et pour fournir un signal IG (145) indiquant l'impédance électrique de la partie du corps ; et
- un capteur de photopléthysmographie, capteur PPG (150), configuré pour mesurer des variations du volume sanguin dans des tissus de la partie du corps et pour fournir un signal PPG (155) indiquant des variations pulsatiles du volume sanguin, telles que le débit sanguin artériel, le rythme cardiaque, la saturation en oxygène du sang ou l'état de perfusion global de la partie du corps.

3. Dispositif (100) de la revendication 1 ou 2, dans lequel l'unité de traitement de signal (160) comprend une unité de corps (162) et au moins une ressource de calcul séparée (164), et l'unité de corps (162) comprend une unité de communication d'unité de corps (170) configurée pour communiquer avec une unité de communication de ressource de calcul (180) de la ressource de calcul (164).

4. Dispositif (100) de la revendication 3, dans lequel la ressource de calcul (164) est configurée pour effectuer entièrement ou partiellement conjointement l'analyse spécifique à la source de signal en combinant les informations des signaux PG (115) avec une information de placement respectif où les capteurs PG (110) sont disposés sur la partie du corps et les informations du signal BIO (125).

5. Dispositif (100) de l'une quelconque des revendications précédentes, dans lequel tout ou partie des capteurs PG (110) et du capteur BIO (120) sont connectés sans fil à l'unité de traitement de signal (160) ; dans lequel n'importe lequel desdits capteurs (110, 120) comprend en outre une unité de communication de capteur (190) configurée pour obtenir n'importe lequel des signaux respectifs (115, 125) des capteurs (110, 120) et pour transmettre lesdits signaux (115, 125) à l'unité de traitement de signal (160).

6. Dispositif (100) de l'une quelconque des revendications précédentes, dans lequel le dispositif (100) est un dispositif portable.

7. Dispositif (100) de l'une quelconque des revendications précédentes, dans lequel l'analyse spécifique à la source de signal comprend une analyse spécifique à la position qui fournit une analyse spécifique au lieu d'une position spécifique dans la partie du corps.

8. Dispositif (100) de l'une quelconque des revendications précédentes, dans lequel l'analyse spécifique à la source de signal comprend une séparation de signal qui fournit au moins un signal (115, 125) qui comprend moins de composants de signaux d'autres sources de signaux.

9. Dispositif (100) de l'une quelconque des revendications précédentes, dans lequel l'analyse spécifique à la source de signal comprend une suppression de bruit qui fournit au moins un signal (115, 125) ayant un composant de signal plus fort d'au moins une source de signal.

10. Dispositif (100) de l'une quelconque des revendications précédentes, dans lequel l'analyse spécifique à la source de signal comprend un diagnostic différentiel qui fournit un diagnostic par rapport aux informations différentielles de plusieurs signaux (115, 125).

11. Dispositif (100) de l'une quelconque des revendications précédentes, dans lequel la partie du corps est un cœur de l'objet, et les multiples capteurs PG (110) sont disposés de telle sorte qu'au moins deux des quatre zones d'auscultation de cœur principales sont couvertes.

12. Dispositif (100) de la revendication 10, au moins quatre des capteurs PG multiples (110) étant disposés de manière à couvrir toutes les quatre zones d'auscultation de cœur principales.

13. Dispositif (100) de l'une quelconque des revendications 1 à 10, dans lequel la partie du corps est le poumon de l'objet.

14. Dispositif (100) de l'une quelconque des revendications 1 à 10, dans lequel la partie du corps est une articulation de l'objet.

15. Procédé pour effectuer conjointement une analyse spécifique à la source de signal dans un dispositif de l'une quelconque des revendications précédentes, comprenant la combinaison des informations de signaux PG (115) avec leurs informations de placement respectif et les informations d'un signal BIO (125).
